# EUROPEAN PATENT APPLICATION

(11) **EP 0 819 430 A1**
(43) Date of publication of application: **21.01.1998**
(21) Application number: 97305348.1
(22) Date of filing: 17.07.1997
(51) Int. Cl.: A61K 31/335, A61K 31/00

(54) **Inhibitor of tumor metastasis or recurrence**

(30) Priority: 17.07.1996 JP 187831/96
(71) Applicant: TAKEDA CHEMICAL INDUSTRIES, LTD., Chuo-ku, Osaka 541 (JP)
(72) Inventor: Sudo, Katsuichi, Takatsuki, Osaka 569 (JP); Houkan, Takashi, Takarazuka, Hyogo 665 (JP)
(74) Representative: Dowden, Marina

(57) **Abstract**

A pharmaceutical composition comprising an angiogenesis inhibitor for inhibition of tumor metastasis or recurrence.

## Description

### Field of the invention

The present invention relates to a pharmaceutical composition for inhibition of cancer metastasis or recurrence comprising an angiogenesis inhibitor, and use thereof.

### Background of the invention

In cancer treatment with chemotherapeutic agents, high-dose or long-term repeated administration is subject to limitation due to the strong toxicity of said agents; conventional treatment with chemotherapeutic agents does not ensure satisfactory effects in terms of inhibition of cancer metastasis and recurrence and the extention of patient survival. There is therefore a great need for the development of drugs and therapies permitting long-term administration and showing excellent therapeutic effects in the inhibition of cancer metastasis and recurrence.

The processes involved in cancer metastasis, include cancer cell release from the primary tumor lesion, their entry and migration in blood vessels, adhesion to the vascular basal membrane, exit from blood vessels, proliferation in metastatic organs and so on. In this respect, cancer metastasis inhibitors differ from the type of anticancer agents that directly inhibit cancer cell proliferation or kill cancer cells, in that they inhibit one of these processes [Bioscience Series, "Gan no Akuseika to Teni," edited by Motoharu Seiki, Chugai Igakusha, 1993].

On the other hand, Japanese Patent Unexamined Publication Nos. 7270/1991 and 7271/1991 disclose that a fumagillol derivative possessing angiogenesis-inhibiting activity is useful as an anticancer agent. Japanese Patent Unexamined Publication Nos. 297469/1992 and 228202/1994 disclose a complex of a fumagillol derivative and cyclodextrin. Japanese Patent Unexamined Publication No. 149634/1995 discloses an antitumor agent consisting of a fumagillol derivative and a platinum complex. Japanese Patent Unexamined Publication No. 242544/1995 discloses an antitumor agent consisting of a fumagillol derivative and a metabolic antagonist.

There is a need for the development of a pharmaceutical that can be safely administered for a long period of time with little burden on cancer patients and that possesses excellent cancer metastasis and recurrence-inhibiting activity.

### Summary of the invention

In view of the above problems, the present inventors made extensive investigations, and found that by using, in tumor treatment, a mildly acting angiogenesis inhibitor, that can be used for a long period of time, of a type different from that of conventional chemotherapeutic agents, tumor metastasis and recurrence-preventing and survival-extending effects can be obtained. The present inventors made further investigations based on this finding, and accomplished the present invention.

Accordingly, the present invention relates to:
(1) use of an angiogenesis inhibitor for the manufacture of a medicament for inhibiting tumor recurrence,
(2) the use according to (1) above, wherein the angiogenesis inhibitor is a fumagillol derivative or a salt thereof,
(3) the use according to (2) above, wherein the fumagillol derivative is a compound represented by the formula: 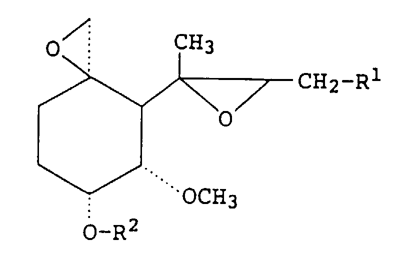 wherein R¹ represents a hydrocarbon group which may be substituted; R² represents a hydrocarbon group which may be substituted or an acyl group which may be substituted,
(4) the use according to (3) above, wherein R¹ is an aliphatic hydrocarbon group which may be substituted,
(5) the use according to (3) above, wherein R¹ is an alkyl group which may be substituted or an alkenyl group which may be substituted,
(6) the use according to (3) above, wherein R¹ is a 2-methylpropyl group which may be substituted or a 2-methyl-1-propenyl group which may be substituted,
(7) the use according to (3) above, wherein R² is an acyl group which may be substituted,
(8) the use according to (3) above, wherein R² is a carbamoyl group which may be substituted,
(9) the use according to (3) above, wherein R¹ is an alkyl group which may be substituted or an alkenyl group which may be substituted and wherein R² is an acyl group which may be substituted,
(10) the use according to (9) above, wherein R² is an alkanoyl group which may be substituted, an alloyl group which may be substituted, a heterocyclic carbonyl group which may be substituted, a carbamoyl group which may be substituted or a thiocarbamoyl group which may be substituted,
(11) the use according to (9) above, wherein R¹ is a 2-methylpropyl group or a 2-methyl-1-propenyl group,
(12) the use according to (9) above, wherein R² is a carbamoyl group which may be substituted,
(13) the use according to (9) above, wherein R² is a carbamoyl group substituted by monochloroacetyl or dichloroacetyl,
(14) the use according to (1) above, wherein the angiogenesis inhibitor is 6-O-(N-chloroacetylcarbamoyl)fumagillol or a salt thereof,
(15) a pharmaceutical composition comprising an angiogenesis inhibitor and cyclophosphamide,
(16) use of a combination of an angiogenesis inhibitor and cyclophosphamide for the manufacture of a medicament for use in combinational therapy for inhibiting tumor metastasis or recurrence,
(17) the use according to (16) above, which is characterized by the additional combination with cisplatin and/or fluorouracil,
(18) use of a combination of an angiogenesis inhibitor and cisplatin for the manufacture of a medicament for inhibiting tumor recurrence,
(19) use of an angiogenesis inhibitor for the manufacture of a medicament for tumor treatment characterized by repeated administration of said angiogenesis inhibitor following administration of one or more tumor chemotherapeutic agents,
(20) the use according to (18) above, wherein the tumor chemotherapeutic agent is cyclophosphamide,
(21) use of an angiogenesis inhibitor for the manufacture of a medicament for tumor treatment characterized by administration of said angiogenesis inhibitor following surgery, radiotherapy, thermotherapy, cryotherapy and/or laser cauterization therapy,
(22) the use according to (21) above intended for long-term repeated administration,
(23) use of an angiogenesis inhibitor for the manufacture of a medicament for enhancement of the antitumor activity, tumor metastasis-inhibiting activity or tumor recurrence-inhibiting activity of another tumor chemotherapeutic agent,
(24) use of 6-O-(N-chloroacetylcarbamoyl)fumagillol or a salt thereof for the manufacture of a medicament for use in the treatment of tumors selected from tumor of brain, tumor of breast, tumor of uterine cervix, tumor of pancreas, tumor of kidney, leukemia, tumor of colon, tumor of kaposi sarcoma, tumor of melanoma, and
(25) use of 6-O-(N-chloroacetylcarbamoyl)fumagillol or a salt thereof for the manufacture of a medicament for use in treating a tumor by inhibiting tumor metastasis to brain.

### Detailed description of the invention

Angiogenesis inhibitors useful in the present invention include, for example, (1) fumagillol derivatives, (2) polysaccharides, (3) eponemycin, (4) pentosan polysulfate, (5) replistatin, (6) DS-4152 (Tecogalan), (7) laminin fragments, (8) antibodies against VEGF receptors containing FLK-1/KDR, such as DC101, (9) antibody against VEGF (Vascular Endothelial Growth Factor), (10) collagenase inhibitors such as marimastat, (11) angiostatin, (12) endostatin and (13) PF-4 (platelet Factor-4) and derivatives thereof, of which fumagillol derivatives etc. are commonly used.

Such "fumagillol derivatives" include, for example, a compound represented by formula [I]: 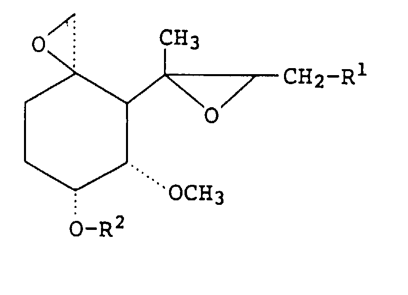 wherein R¹ represents a hydrocarbon group which may be substituted; R² represents a hydrocarbon group which may be substituted or an acyl group which may be substituted, or a salt thereof.

With respect to formula [I] above, the "hydrocarbon group" in the "hydrocarbon group which may be substituted" represented by R¹ or R² is exemplified by aliphatic hydrocarbon groups and cyclic hydrocarbon groups. Such "aliphatic hydrocarbon groups" include, for example, aliphatic hydrocarbon groups having 1 to 20 carbon atoms (e.g., alkyl groups, alkenyl groups, alkynyl groups). Such "cyclic hydrocarbon groups" include, for example, cyclic hydrocarbon groups having 3 to 20 carbon atoms (e.g., cycloalkyl groups, cycloalkenyl groups, aryl groups).

Preferable examples of such "alkyl groups" include C₁₋₁₀ alkyl groups such as methyl, ethyl, propyl, 2-propyl, 1-ethylpropyl, butyl, 1-methylpropyl, 2-methylpropyl, 1,1-dimethylethyl, 1,1-dimethylbutyl, 2,2-dimethylbutyl, pentyl, 3-methylbutyl, 2,2-dimethylpropyl and hexyl.

Preferable examples of such "alkenyl groups" include C₂₋₁₀ alkenyl groups such as ethenyl, 2-propenyl, 1-methylethenyl, butenyl, 2-methyl-1-propenyl, 2-methyl-2-propenyl, 1-methyl-2-propenyl, 2-pentenyl, 3-pentenyl, 4-pentenyl, 2-hexenyl, 3-hexenyl and 5-hexenyl.

Preferable examples of such "alkynyl groups" include C₂₋₁₀ alkynyl groups such as ethynyl, 2-propynyl, 2-butyn-1-yl, 3-butyn-2-yl, 1-pentyn-3-yl, 3-pentyn-1-yl, 4-pentyn-2-yl and 3-hexyn-2-yl.

Preferable examples of such "cycloalkyl groups" include C₃₋₁₀ cycloalkyl groups such as cyclopropyl, cyclobutyl, cyclopentyl and cyclohexyl.

Preferable examples of such "cycloalkenyl groups" include C₃₋₁₀ cycloalkenyl groups such as cyclobutenyl, cyclopentenyl, cyclohexenyl and cyclohexadienyl.

Preferable examples of such "aryl groups" include C₆-₁₄ aryl groups such as phenyl, 1-naphthyl and 2-naphthyl.

The "hydrocarbon group" represented by R¹ is preferably exemplified by aliphatic hydrocarbon groups having 1 to 20 carbon atoms (e.g., alkyl groups, alkenyl groups). More preferable examples include 2-methylpropyl groups and 2-methyl-1-propenyl groups.

Regarding the "hydrocarbon group" represented by R², aliphatic hydrocarbon groups having 1 to 20 carbon atoms (e.g., alkyl groups, alkenyl groups) are commonly used.

With respect to formula [I] above, the "acyl group" of the "acyl group which may be substituted" represented by R² is exemplified by alkanoyl groups which may be substituted, alloyl groups which may be substituted, heterocyclic carbonyl groups which may be substituted, carbamoyl groups which may be substituted, thiocarbamoyl groups which may be substituted, alkylsulfonyl groups which may be substituted, arylsulfonyl groups which may be substituted, sulfamoyl groups which may be substituted, alkoxycarbonyl groups which may be substituted and aryloxycarbonyl groups which may be substituted. Of these groups, alkanoyl groups which may be substituted, alloyl groups which may be substituted, heterocyclic carbonyl groups which may be substituted, carbamoyl groups which may be substituted, thiocarbamoyl groups which may be substituted, etc. are preferred, carbamoyl groups which may be substituted are most preferred.

Such "alkanoyl groups" of "alkanoyl groups which may be substituted" include, for example, C₁₋₂₀ alkanoyl groups (e.g., formyl, acetyl, propionyl, isopropionyl, butyryl, pentanoyl, hexanoyl, heptanoyl, octanoyl, nonanoyl, lauroyl, undecanoyl, myristoyl, palmitoyl, stearoyl), C₁₋₆ alkanoyl groups (e.g., formyl, acetyl, propionyl, isopropionyl, butyryl, pentanoyl, hexanoyl).

Such "alloyl groups" of "alloyl groups which may be substituted" include, for example, C₇₋₁₆ alloyl groups (e.g., benzoyl, 1-naphthoyl, 2-naphthoyl).

Such "heterocyclic carbonyl groups" of "heterocyclic carbonyl groups which may be substituted" include, for example, 5- or 6-membered heterocyclic carbonyl groups containing 1 to 4 hetero atoms (e.g., nitrogen, oxygen, sulfur) in addition to carbon atoms (e.g., 3-pyrrolylcarbonyl, 2-imidazolylcarbonyl, 1-pyrazolylcarbonyl, 3-isothiazolylcarbonyl, 3-isoxazolylcarbonyl, pyrazinylcarbonyl, 2-pyrimidinylcarbonyl, 3-pyrazinylcarbonyl, 2-indolizinylcarbonyl, 2-isoindolylcarbonyl, 1-indolylcarbonyl, 2-froyl, 2-thenoyl, nicotinoyl, isonicotinoyl, morpholinocarbonyl, piperidinocarbonyl, piperazinocarbonyl).

Such "alkylsulfonyl groups" of "alkylsulfonyl groups which may be substituted" include, for example, C₁₋₂₀ alkylsulfonyl groups (e.g., methylsulfonyl, ethylsulfonyl, propylsulfonyl, isopropylsulfonyl), C₁₋₆ alkylsulfonyl groups (e.g., methylsulfonyl, ethylsulfonyl, propylsulfonyl).

Such "carbamoyl groups" of "carbamoyl groups which may be substituted" include carbamoyl groups, mono-substitutional carbamoyl groups and di-substitutional carbamoyl groups, substituents therefor including C₁₋₆ alkyl groups (e.g., methyl, ethyl, propyl, isopropyl, butyl, isobutyl), C₆₋₁₄ aryl groups (e.g., phenyl, 1-naphthyl, 2-naphthyl), C₇₋₁₆ aralkyl groups (e.g., benzyl), C₁₋₆ alkanoyl groups (e.g., acetyl, propionyl, isopropionyl, butyryl), C₇₋₁₆ alloyl groups (e.g., benzoyl, 1-naphthoyl, 2-naphthoyl), 5- or 6-membered heterocyclic carbonyl groups (e.g., 3-pyrrolylcarbonyl, 2-imidazolylcarbonyl, 1-pyrazolylcarbonyl, 3-isothiazolylcarbonyl, 3-isoxazolylcarbonyl, pyrazinylcarbonyl, 2-pyrimidinylcarbonyl, 3-pyrazinylcarbonyl, 2-indolizinylcarbonyl, 2-isoindolylcarbonyl, 1-indolylcarbonyl, 2-froyl, 2-thenoyl, nicotinoyl, isonicotinoyl, morpholinocarbonyl, piperidinocarbonyl, piperazinocarbonyl). Preferable examples of such "carbamoyl groups which may be substituted" include mono- or di-C₁₋₆ alkanoyl-carbamoyl groups (e.g., acetylcarbamoyl).

Such "thiocarbamoyl groups" of "thiocarbamoyl groups which may be substituted" include thiocarbamoyl groups, mono-substitutional thiocarbamoyl groups and di-substitutional thiocarbamoyl groups, substituents therefor including C₁₋₆ alkyl groups (e.g., methyl, ethyl, propyl, isopropyl, butyl, isobutyl), C₆₋₁₄ aryl groups (e.g., phenyl, 1-naphthyl, 2-naphthyl), C₇₋₁₆ aralkyl groups (e.g., benzyl), C₁₋₆ alkanoyl groups (e.g., acetyl, propionyl, isopropionyl, butyryl), C₇₋₁₆ alloyl groups (e.g., benzoyl, 1-naphthoyl, 2-naphthoyl), 5- or 6-membered heterocyclic carbonyl groups (e.g., 3-pyrrolylcarbonyl, 2-imidazolylcarbonyl, 1-pyrazolylcarbonyl, 3-isothiazolylcarbonyl, 3-isoxazolylcarbonyl, pyrazinylcarbonyl, 2-pyrimidinylcarbonyl, 3-pyrazinylcarbonyl, 2-indolizinylcarbonyl, 2-isoindolylcarbonyl, 1-indolylcarbonyl, 2-froyl, 2-thenoyl, nicotinoyl, isonicotinoyl, morpholinocarbonyl, piperidinocarbonyl, piperazinocarbonyl). Preferable examples of such "thiocarbamoyl groups which may be substituted" include mono- or di-C₁₋₆ alkanoyl-thiocarbamoyl groups (e.g., acetylthiocarbamoyl).

Such "arylsulfonyl groups" of "arylsulfonyl groups which may be substituted" include, for example, C₆₋₁₄ arylsulfonyl groups (e.g., benzenesulfonyl, 1-naphthylsulfonyl, 2-naphthylsulfonyl).

Such "sulfamoyl groups" of "sulfamoyl groups which may be substituted" include sulfamoyl groups, mono-substitutional sulfamoyl groups and di-substitutional sulfamoyl groups, substituents therefor including C₁₋₆ alkyl groups (e.g., methyl, ethyl, propyl, isopropyl, butyl, isobutyl), C₆₋₁₄ aryl groups (e.g., phenyl, 1-naphthyl, 2-naphthyl) and C₇₋₁₆ aralkyl groups (e.g., benzyl).

Such "alkoxycarbonyl groups" of "alkoxycarbonyl groups which may be substituted" include, for example, C₁₋₂₀ alkoxy-carbonyl groups (e.g., methoxycarbonyl, ethoxycarbonyl, propoxycarbonyl, isopropoxycarbonyl, butoxycarbonyl, isobutoxycarbonyl), C₁₋₆ alkoxy-carbonyl groups (e.g., methoxycarbonyl, ethoxycarbonyl, propoxycarbonyl) etc. being commonly used.

Such "aryloxycarbonyl groups" of "aryloxycarbonyl groups which may be substituted" include C₆₋₁₄ aryloxy-carbonyl groups (e.g., phenoxycarbonyl, 1-naphthyloxycarbonyl, 2-naphthyloxycarbonyl).

With respect to formula [I] above, substituents which may be present in the "hydrocarbon group which may be substituted" and "acyl group which may be substituted" represented by R¹ or R² include, for example, but are not limited to, amino group, mono- or di-C₁₋₆ alkylamino groups (e.g., methylamino, ethylamino, propylamino, isopropylamino, dimethylamino, diethylamino), mono- or di-C₆₋₁₀ arylamino groups (e.g., phenylamino, diphenylamino), mono- or di-C₇₋₁₁ aralkylamino groups (e.g., benzylamino, dibenzylamino), azide group, nitro group, halogens (e.g., fluorine, chlorine, bromine, iodine), hydroxy group, C₁₋₆ alkoxy groups (e.g., methoxy, ethoxy, propoxy, isopropoxy, butoxy), C₆₋₁₀ aryloxy groups (e.g., phenoxy, 1-naphthyloxy, 2-naphthyloxy), C₇₋₁₁ aralkyloxy groups (e.g., benzyloxy), formyloxy group, C₁₋₆ alkyl-carbonyloxy groups (e.g., acetoxy, propionyloxy), C₆₋₁₀ aryl-carbonyloxy groups (e.g., benzoyloxy), C₇₋₁₁ aralkyl-carbonyloxy groups (e.g., benzylcarbonyloxy), sulfonyloxy group, C₁₋₆ alkylsulfonyloxy groups (e.g., methylsulfonyloxy), mercapto group, C1-6 alkylthio groups (e.g., methylthio, ethylthio, propylthio, isopropylthio), C₆₋₁₀ arylthio groups (e.g., phenylthio, 1-naphthylthio, 2-naphthylthio), C₇₋₁₁ aralkylthio groups (e.g., benzylthio), phosphonooxy group, cyano group, carbamoyl group, mono- or di-C₁₋₆ alkylcarbamoyl groups (e.g., methylcarbamoyl, ethylcarbamoyl, dimethylcarbamoyl, diethylcarbamoyl), mono- or di-C₆₋₁₀ arylcarbamoyl groups (e.g., phenylcarbamoyl, diphenylcarbamoyl), mono- or di-C₇₋₁₁ aralkylcarbamoyl groups (e.g., benzylcarbamoyl, dibenzylcarbamoyl), carboxyl group, C₁₋₆ alkoxy-carbonyl groups (e.g., methoxycarbonyl, ethoxycarbonyl), C₆₋₁₀ aryloxy-carbonyl groups (e.g., phenoxycarbonyl, l-naphthyloxycarbonyl, 2-naphthyloxycarbonyl), C₇₋₁₁ aralkyloxy-carbonyl groups (e.g., benzyloxycarbonyl), formyl group, C₁₋₆ alkyl-carbonyl groups which may be substituted by one to three halogenes (e.g., acetyl, propionyl, isopropionyl, butyryl, pentanoyl, hexanoyl), C₆₋₁₀ aryl-carbonyl groups (e.g., benzoyl, 1-naphthoyl, 2-naphthoyl), C₇₋₁₁ aralkyl-carbonyl groups (e.g., benzylcarbonyl), sulfo group, C1-6 alkylsulfinyl groups (e.g., methylsulfinyl, ethylsulfinyl), C₆₋₁₀ arylsulfinyl groups (e.g., benzenesulfinyl, 1-naphthylsulfinyl, 2-naphthylsulfinyl), C₁₋₆ alkylsulfonyl groups (e.g., methylsulfonyl, ethylsulfonyl), C₆₋₁₀ arylsulfonyl groups (e.g., benzenesulfonyl, 1-naphthylsulfonyl, 2-naphthylsulfonyl), C₁₋₆ alkyl groups (e.g., methyl, ethyl, propyl, isopropyl, butyl, isobutyl, sec-butyl), C₂₋₆ alkenyl groups (e.g., vinyl, allyl, 2-butenyl), C₂₋₆ alkynyl groups (e.g., ethynyl, propargyl), C₃₋₆ cycloalkyl groups (e.g., cyclopropyl, cyclobutyl, cyclopentyl, cyclohexyl), C₃₋₆ cycloalkenyl groups (e.g., cyclobutenyl, cyclopentenyl, cyclohexenyl, cyclohexadienyl), C₆₋₁₀ aryl groups (e.g., phenyl, 1-naphthyl, 2-naphthyl), mono- to tri-cyclic heterocyclic groups (e.g., heterocyclic groups formed by 1 to 3 5- or 6-membered rings containing 1 to 4 hetero atoms selected from nitrogen, oxygen and sulfur: pyridyl, pyrazyl, pyrimidyl, quinolyl, isoquinolyl, indolyl, isoindolyl, indazolyl, pyridazinyl, imidazolyl, pyrazolyl, pyrrolyl, furyl, benzofuranyl, thienyl, benzothienyl, benzimidazolyl, quinazolyl, pyrrolidinyl, pyrrolinyl, imidazolizinyl, imidazolinyl, pyrazolidinyl, pyrazolinyl, piperidyl, piperazinyl, indolidyl, isoindolidyl, morpholinyl etc.), and mono- to tri-cyclic heterocyclic thio groups (e.g., groups resulting from binding of a thio group to the above-mentioned heterocyclic groups, specifically 4-pyridylthio, 2-pyridylthio, 1,3,4-thiadiazol-2-ylthio, 5-tetrazolylthio, 2-benzothiazolylthio, 8-quinolylthio etc.). These substituents may be present on "hydrocarbon groups" and "acyl groups," as long as they are chemically acceptable, the number thereof being 1 to 5, preferably 1 to 3. When there are 2 or more substituents, they may be identical or not. As long as they are chemically acceptable, these substituents may have 1 to 3 substituents selected from amino group, mono- or di-C₁₋₆ alkylamino groups, nitro group, halogens, hydroxy group, C₁₋₆ alkoxy groups, C₁₋₆ alkyl-carbonyloxy groups, sulfonyloxy group, C₁₋₆ alkylsulfonyloxy groups, mercapto groups, C₁₋₆ alkylthio groups, phosphonooxy group, cyano group, carbamoyl group, mono- or di-C₁₋₆ alkylcarbamoyl groups, carboxyl group, C₁₋₆ alkoxy-carbonyl groups, formyl group, C₁₋₆ alkyl-carbonyl groups, sulfo group, C₁₋₆ alkylsulfinyl groups etc.

With respect to formula [I] above, R¹ represents a hydrocarbon group which may be substituted, preferably an alkyl group which may be substituted or an alkenyl group which may be substituted, and a 2-methyl-1-propenyl group which may be substituted or a 2-methylpropyl group which may be substituted is commonly used. Preferable examples of R¹ include 2-methyl-1-propenyl or 2-methylpropyl groups which may be substituted by hydroxy group, di-C₁₋₆ alkylamino groups (e.g., dimethylamino) etc. The non-substitutional 2-methyl-1-propenyl group and 2-methylpropyl group are the most commonly used groups for R¹.

With respect to formula [I] above, R² represents a hydrocarbon group which may be substituted or an acyl group which may be substituted, preferably an acyl group which may be substituted, with greater preference given to alkanoyl groups which may be substituted, alloyl groups which may be substituted, heterocyclic carbonyl groups which may be substituted, carbamoyl groups which may be substituted, thiocarbamoyl groups which may be substituted, etc. Among them carbamoyl groups which may be substituted, is preferred.

Preferable examples of R¹ and R² in formula [I] above include (A) through (N) below.
(A) R¹ is an aliphatic hydrocarbon group which may be substituted.
(B) R¹ is an alkyl group which may be substituted or an alkenyl group which may be substituted.
(C) R¹ is a 2-methylpropyl group which may be substituted or a 2-methyl-1-propenyl group which may be substituted.
(D) R¹ is a 2-methylpropyl group or a 2-methyl-1-propenyl group.
(E) R² is an acyl group which may be substituted.
(F) R² is an alkanoyl group which may be substituted, an alloyl group which may be substituted, a heterocyclic carbonyl group which may be substituted, a carbamoyl group which may be substituted or a thiocarbamoyl group which may be substituted.
(G) R² is a carbamoyl group which may be substituted.
(H) R² is a mono-C₁₋₆ alkanoyl-carbamoyl group which may be substituted.
(I) R² is a mono-C₁₋₆ alkanoyl-carbamoyl group which may have 1 to 3 halogens.
(J) R² is a carbamoyl group substituted by monochloroacetyl or dichloroacetyl.
(K) R² is a carbamoyl group substituted by a C₁₋₆ alkyl group.
(L) R² is a mono-C₁₋₆ alkyl-carbamoyl group.
(M) The compound represented by formula [I] is 6-O-(N-chloroacetylcarbamoyl)fumagillol or a salt thereof.
(N) The compound represented by formula [I] is 6-O-(N-methylcarbamoyl)fumagillol or a salt thereof.

Of the various angiogenesis inhibitors, the fumagillol derivative represented by formula [I] above, for example, can be produced using the methods described in Japanese Patent Unexamined Publication Nos. 7270/1991 and 7271/1991, those based thereon, and other methods.

When the fumagillol derivative represented by formula [I] above contains a basic group, it can be obtained as a pharmaceutically acceptable acid adduct salt by a commonly known method or a method based thereon. Such acids include, for example, inorganic acids (e.g., hydrochloric acid, sulfuric acid, phosphoric acid, hydrobromic acid), organic acids (e.g., acetic acid, trifluoroacetic acid, succinic acid, maleic acid, fumaric acid, propionic acid, citric acid, tartaric acid, malic acid, lactic acid, oxalic acid, methanesulfonic acid, p-toluenesulfonic acid) and amino acids (e.g., glutamic acid, aspartic acid). When the fumagillol derivative contains an acidic group, it can be converted to a salt with a pharmaceutically acceptable base by a commonly known method or a method based thereon. Such bases include, for example, alkali metals (e.g., sodium, potassium), alkaline earth metals (e.g., calcium, magnesium), organic bases (e.g., trimethylamine, triethylamine, pyridine, piperidine, ethanolamine), aluminum and ammonium.

The pharmaceutical composition comprising an angiogenesis inhibitor such as fumagillol derivative of the present invention is effective and permits long-term administration in the prevention and/or treatment of various diseases (e.g., tumor recurrence and growth of metastatic colony) in mammals (e.g., humans, bovines, horses, dogs, cats, monkeys, mice, rats, especially humans) because it is low in toxicity and prevalence of adverse reactions.

The pharmaceutical composition comprising an angiogenesis inhibitor and cyclophosphamide of the present invention is effective for inhibiting tumor recurrence and formation and growth of metastatic colony, which is also effective for extending the survival of tumor patients.

The pharmaceutical composition comprising an angiogenesis inhibitor and cisplatin of the present invention is effective for inhibiting tumor recurrence and growth of metastatic colony.

The pharmaceutical composition comprising (1) an angiogenesis inhibitor and interleukin or (2) a complex of fumagillol with a highly water-soluble cyclodextrin derivative of the present invention is effective for inhibiting tumor recurrence and growth of metastatis colony.

Although the above-described "angiogenesis inhibitor" may be used in the form of bulk powder as such, it is normally administered in a preparation prepared by a conventional method, using appropriate amounts of carriers for pharmaceutical compositions as described below. Such "carriers for pharmaceutical compositions" include, for example, excipients (e.g., calcium carbonate, kaolin, sodium hydrogen carbonate, lactose, D-mannitol, starch, crystalline cellulose, talc, granular sugar, porous substances), binders (e.g., rubbers, glutenized starch, gelatin, hydroxypropyl cellulose, hydroxypropylmethyl cellulose, pullulan), thickening agents (e.g., natural rubbers, cellulose derivatives, acrylic acid derivatives), disintegrants (e.g., carboxymethyl cellulose calcium, croscarmellose sodium, crospovidone, low-substitutional hydroxypropyl cellulose, partially glutenized starch), solvents (e.g., water for injection, alcohols, propylene glycol, macrogol, sesame oil, corn oil), dispersing agents (e.g., Tween 80, HCO60, polyethylene glycol, carboxymethyl cellulose, sodium alginate), dissolution aids (e.g., polyethylene glycol, propylene glycol, D-mannitol, benzyl benzoate, ethanol, trisaminomethane, triethanolamine, sodium carbonate, sodium citrate), suspending agents (e.g., stearyltriethanolamine, sodium lauryl sulfate, benzalkonium chloride, polyvinyl alcohol, polyvinylpyrrolidone, hydroxymethyl cellulose), soothing agents (e.g., benzyl alcohol), isotonizing agents (e.g., sodium chloride, glycerol), buffers (e.g., phosphates, acetates, carbonates, citrates), lubricants (e.g., magnesium stearate, calcium stearate, talc, starch, sodium benzoate), coloring agents (e.g., tar pigments, caramel, iron sesquioxide, titanium oxide, riboflavins), correctives (e.g., sweeteners, flavors), stabilizers (e.g., sodium sulfite, ascorbic acid), preservatives (e.g., parabens, sorbic acid), biodegradable high-molecular polymers (e.g., lactic acid-glycolic acid copolymer, lactic acid-butyric acid copolymer), cyclodextrins (e.g., maltosyl-β-cyclodextrin, maltosyl-β-cyclodextrincarboxylic acid), sodium hydroxide and MIGLYOL.

Also, to mitigate adverse reactions, reducing agents (e.g., vitamin C), steroidal anti-inflammatory drugs, non-steroidal anti-inflammatory drugs etc. may be used.

The angiogenesis inhibitor comprised in the pharmaceutical composition of the present invention is comprised in an amount effective to treat and prevent the diseases shown below, normally at about 0.1 to about 100% by weight of the entire preparation. Also, the pharmaceutical composition used in the present invention may contain pharmaceutically active ingredients (e.g., the antitumor agents shown below), in addition to the above-described "angiogenesis inhibitor" or a pharmaceutically acceptable salt thereof; these ingredients are not subject to limitation, as long as the object of the present invention is accomplished, permitting the use as a composite agent comprising them in appropriate ratios. Useful dosage forms include, for example, tablets (including sugar-coated tablets and film-coated tablets), pills, capsules (including microcapsules and enteric capsules), granules, fine granules, powders, eyedrops, drip infusions, syrups, emulsions, suspensions, injectable preparations, inhalations, ointments, suppositories, troches, poultices and sustained-release preparations. These preparations are prepared by conventional methods (e.g., the method described in the Pharmacopoeia of Japan, 12th edition).

Production methods for principal preparations of the present invention are described below but are not to be construed as limiting.

### (1) Tablets

An angiogenesis inhibitor or a salt thereof, as such or in the form of a uniformly kneaded mixture with an excipient, a binder, a disintegrant or another appropriate additive, is granulated by an appropriate method, after which it is subjected to compressive molding in the presence of a lubricant etc. The resulting tablet may be coated with an appropriate coating agent for taste masking, enteric solubility, sustained release or another purpose.

### (2) Injectable preparation

A given amount of an angiogenesis inhibitor or a salt thereof is dissolved, suspended or emulsified in water for injection etc., in the presence of a stabilizer, a dissolution aid, a suspending agent, an emulsifier, a buffer, a preservative, cyclodextrin, sodium hydroxide etc. added as necessary, to a given dosing volume.

### (3) Suppository

An angiogenesis inhibitor or a salt thereof is uniformly mixed with an oil-soluble base, a water-soluble base or another appropriate base substance in the presence of an emulsifier, a suspending agent etc. added as necessary, after which it is molded into an appropriate shape.

### (4) Capsular preparation

An angiogenesis inhibitor or a salt thereof and an appropriate excipient and other additives are uniformly mixed, or granulated by an appropriate method, whether coated with an appropriate coating agent, and the resulting mixture or grains are loosely filled in capsules.

### (5) Eyedrops

An angiogenesis inhibitor or a salt thereof is added to a solvent and dissolved completely.

The pharmaceutical composition of the present invention is low in toxicity and very safe, and is useful as a therapeutic and prophylactic drug for various diseases, e.g., (1) benign and malignant tumors (e.g., stomach cancer, esophageal cancer, duodenal cancer, tongue cancer, pharyngeal cancer, brain tumor, schwannoma, rectal cancer, colon cancer, non-small cell lung cancer, small cell lung cancer, neuronal tissues cancer, liver cancer, kidney cancer, breast cancer, bile duct cancer, pancreatic cancer, prostatic cancer, uterine body cancer, uterine cervical cancer, ovarian cancer, gallbladder cancer, skin cancer, malignant melanoma, thyroid cancer, bone tumor, angioma, angiofibroma, retinal sarcoma, penis cancer, child solid cancers, AIDS-related Kaposi's sarcoma, Kaposi's sarcoma, leukemia, lymphoma), their recurrence and metastasis to other organs, (2) diabetic retinopathy, neovascular glaucoma, rubeosis of iris, trachoma, central retinal vein occlusion, central retinal artery occlusion, retinopathy of prematurity, senile macular degeneration, sickle cell retinopathy, corneal opacity, and other pathologic conditions which cause ischemic, (3) chronic arthritis, rheumatism, rheumatoid arthritis, (4) psoriasis, hypertrophic scars, (5) abnormal capillary network in atherosclerotic lesion external tunica, (6) allergic inflammation, airway inflammation, nephritis, and other chronic inflammation.

The pharmaceutical composition comprising fumagillol derivative such as 6-O-(N-chloroacetylcarbamoyl)fumagillol is especially effective for treating tumors of brain, breast, uterine cervix, pancreas, kidney, leukemia, Kaposi sarcoma, colon, and melanoma.

The pharmaceutical composition of the present invention can be used in combination with other pharmaceutical compositions, as long as the object of the present invention is accomplished. When used as an antitumor agent, for example, the pharmaceutical composition of the present invention exhibits excellent antitumor affects even in use as a single agent; however, its effect can be enhanced by combined use with another drug possessing antitumor activity or antitumor activity-enhancing activity (multiple combinational use). Another advantage of combinational use is that the amounts of drugs used can be reduced, which in turn decreases the prevalence of adverse reactions and significantly contributes to improvements of the patient's quality of life (e.g., mitigation of performance stasis and pain, suppression of edema, promotion of appetite, body weight gain).

Examples of antitumor agents used in composite agents or combinational therapy include, but are not limited to, the agents shown below.
[1] Alkylating agents include, for example, nitrogen mustard, nitrogen mustard-N-oxide hydrochloride, chlorambutyl, cyclophosphamide, ifosfamide, thiotepa, carboquone, improsulfan tosilate, busulfan, nimustine hydrochloride, mitobronitol, melphalan, dacarbazine, ranimustine, estramustine sodium phosphate, triethylenemelamine, carmustine, lomustine, streptozocin, pipobraman, etoglucid, carboplatin, cisplatin, miboplatin, nedaplatin, oxaliplatin, altretamine, ambamustine, dibrospidium hydrochloride, fotemstine, prednismustine, pumitepa, lipomustine, temozolomide, treosulfan, trofosfamide and zinostatin stimalamer.
[2] Metabolic antagonists include, for example, mercaptopurine, Thioinosie, methotrexate, enocitabine, cytarabine, cytarabine ocfosfate, ancytabine hydrochloride, 5-FU drugs (e.g., fluorouracil, tegafur, UFT, doxifluridine, carmofur, Furtulon, Neofurtulon), aminopterin, leucovorin calcium, tabloid, butosin, calcium folinate, calcium levofolinate, cladribine, emitefur, fludarabine, gemcitabine, hydroxycarbamide and pentostatin.
[3] Plant alkaloids include, for example, etoposide, etoposide phosphate, vinblastine sulfate, vincristine sulfate, vindesine sulfate, teniposide, paclitaxel docetaxel and vinorelbine.
[4] Anticancer antibiotics include, for example, actinomycin D, actinomycin C, mitomycin C, chromomycin A₃, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride and mitoxantrone hydrochloride.
[5] Sex hormones include, for example, fosfestrol, diethylstilbestrol, chlorotrianisene, medroxyprogesterone acetate, megestrol acetate, chloromadinone acetate, cyproterone acetate, anti-estrogens (e.g., tamoxifen citrate, toremifene citrate), mepitiostane, testolactone, aminoglutethimide, LH-RH agonists (e.g., goserelin acetate, buserelin, leuprorelin), droloxifene, epitiostanol, ethynylestradiol sulfonate, LH-RH antagonists (e.g., cetrorelix, ganirelix, azaline B), aromatase inhibitors (e.g., fadrozole hydrochloride, anastrozole, retrozole, exemestane, borozole, formestane), anti-androgen drugs (e.g., flutamide, bicalutamide), retinoids, and drugs which retard retinoid metabolism (e.g., riarozole).
[6] Biological response modifiers (BRMs) include, for example, Picibanil, crestin, sizofilan, lentinan, ubenimex, interferons, interleukins, macrophage colony-stimulating factor, granulocyte colony-stimulating factor, erythropoietin, lymphotoxin, BCG vaccine, Corynebacterium parvum, levamisole, polysaccharide K and procodazole.
[7] Other useful antitumor agents include L-asparaginase, aceglatone, procarbazine hydrochloride, doxorubicin, protoporphyrin-cobalt complex salt, mercury hematoporphyrin-sodium, topoisomerase I inhibitors (e.g., camptotecin, topotecan, irinotecan), topoisomerase II inhibitors, differentiation inducers (e.g., retinoids, vitamin D) and growth factor inhibitors (e.g., suramin), taxol and taxotene.

Of these agents, 1 to 5 (preferably 1 to 3) may be selected for combinational use with the pharmaceutical composition of the present invention. The combination, combined dose, duration of administration and method of administration of these agents can be optionally varied during cancer treatment.

In addition, the pharmaceutical composition of the present invention extends disease-free survival, suppresses cancer metastasis or recurrence, prolongs survival and provides other benefits when used after (1) surgery, (2) hypertensive chemotherapy using angiotensin II etc., (3) thermotherapy, (4) cryotherapy, (5) laser cauterization, (6) radiotherapy, etc., or after a treatment comprising 2 or 3 of these therapies.

Also, treatment with the pharmaceutical composition of the present invention can be combined with supportive therapies [e.g., (i) administration of antibiotics (e.g., β-lactams such as pansporin, macrolides such as clarythromycin), (ii) total parenteral nutrition, administration of amino acid preparations and general vitamin preparations for improvement of malnutrition, (iii) morphine administration for pain mitigation, (iv) administration of drugs which mitigate adverse reactions such as nausea, vomiting, anorexia, diarrhea, leukopenia, thrombocytopenia, hemoglobin concentration reduction, hair loss, hepatopathy, renopathy, DIC and fever), (v) administration of drugs for inhibition of multiple drug resistance in cancer].

Preferably, the pharmaceutical composition of the present invention is administered orally (including sustained-release preparations), intravenously (including boluses, infusions and clathrates), subcutaneously and intramuscularly (including boluses, infusions and sustained-release preparations), transdermally, intratumorally or proximally after the above-described treatment is conducted.

Although the content of the compound of the present invention in the above-described pharmaceutical compositions varies depending on the compound chosen, animal species as the subject of administration, dosing frequency etc., efficacy is obtained in a broad range of content. For example, the daily dose of the pharmaceutical composition of the present invention in oral administration to an adult patient with malignant tumor (e.g., breast cancer patient) is normally about 0.001 to about 500 mg/kg body weight, preferably about 0.1 to about 40 mg/kg body weight, and more preferably about 0.5 to about 20 mg/kg body weight. In non-oral administration or combinational use with other anticancer agents or therapies, however, doses are generally lower than those shown above. However, the amount of actually administered compound is determined on the basis of the compound chosen, dosage form, patient age, body weight, sex, severity of disease, route of administration, duration and intervals of administration and other factors, and is optionally variable on discretion of the physician in charge.

The above-described pharmaceutical compositions are not subject to limitation as to route of administration, and can be administered orally or non-orally, for example. The term "non-oral" as used herein is defined as including intravenous administration, intramuscular administration, subcutaneous administration, intranasal administration, intradermal administration, instillation, intracerebral administration, intrarectal administration, intravaginal administration, intraperitoneal administration, intratumoral administration and near-the-tumor administration.

Duration and intervals of administration of the above-described pharmaceutical composition is varied according to various conditions on discretion of the physician in charge, and available methods of administration include administration in portions, administration for consecutive days, intermittent administration, short-term mass administration and repeated administration. In oral administration, for example, the pharmaceutical composition is desirably administered in 1 to several portions (1 to 3 portions, in particular) per day. The pharmaceutical composition can also be used as a sustained-release oral preparation.

### [Examples]

The present invention is hereinafter described in more detail by means of the following working examples, experimental examples etc., which are not to be construed as limitative, and which may be varied, as long as they remain within the scope of the invention.

### Example 1

| | |
|---|---|
| 6-O-(N-chloroacetylcarbamoyl)fumagillol | 100 mg |
| Maltosyl-β-cyclodextrin | 726 mg |
| Sodium hydroxide | 33.3 µg |
| Distilled water for injection | Total volume 5.0 ml |

To distilled water for injection containing 726 mg of maltosyl-β-cyclodextrin dissolved therein, 100 mg of 6-O-(N-chloroacetylcarbamoyl)fumagillol was added. To this mixture, an aqueous solution of sodium hydroxide (33.3 µg) was added to make a total volume of 5 ml, followed by filtration. This filtrate was filled in a vial, after which it was freeze-dried to yield an injectable preparation.

### Example 2

| | |
|---|---|
| 6-O-(N-chloroacetylcarbamoyl)fumagillol | 100 mg |
| MIGLYOL 812 | Total volume 100 ml |

6-O-(N-chloroacetylcarbamoyl)fumagillol was dissolved in MIGLYOL 812 to a concentration of 100 mg/ml to yield a uniform solution.

### Experimental Example 1

A 5% glucose solution of a maltosyl-cyclodextrin clathrate of 6-O-(N-chloroacetylcarbamoyl)fumagillol (hereinafter referred to as compound A) was prepared. Also, an oily solution of compound A in MIGLYOL was prepared and diluted to a concentration of 0.2 ml/mouse. Cyclophosphamide at 150 mg/kg was dissolved in physiological saline.

5 × 10⁻⁵ cells of M5076 mouse reticulum cell sarcoma were intradermally transplanted to 5 (10 for the control group) C57BL/6 female mice (Clea Japan) per group. Five days later, cyclophosphamide at 150 mg/kg was orally administered. An oily solution of compound A in MIGLYOL or a β-cyclodextrin clathrate of compound A was subcutaneously administered once every 6 days, starting 5 days after cyclophosphamide administration. Figures for tumor size are expressed as mean of major and minor diameters of the tumor. The results are shown below.

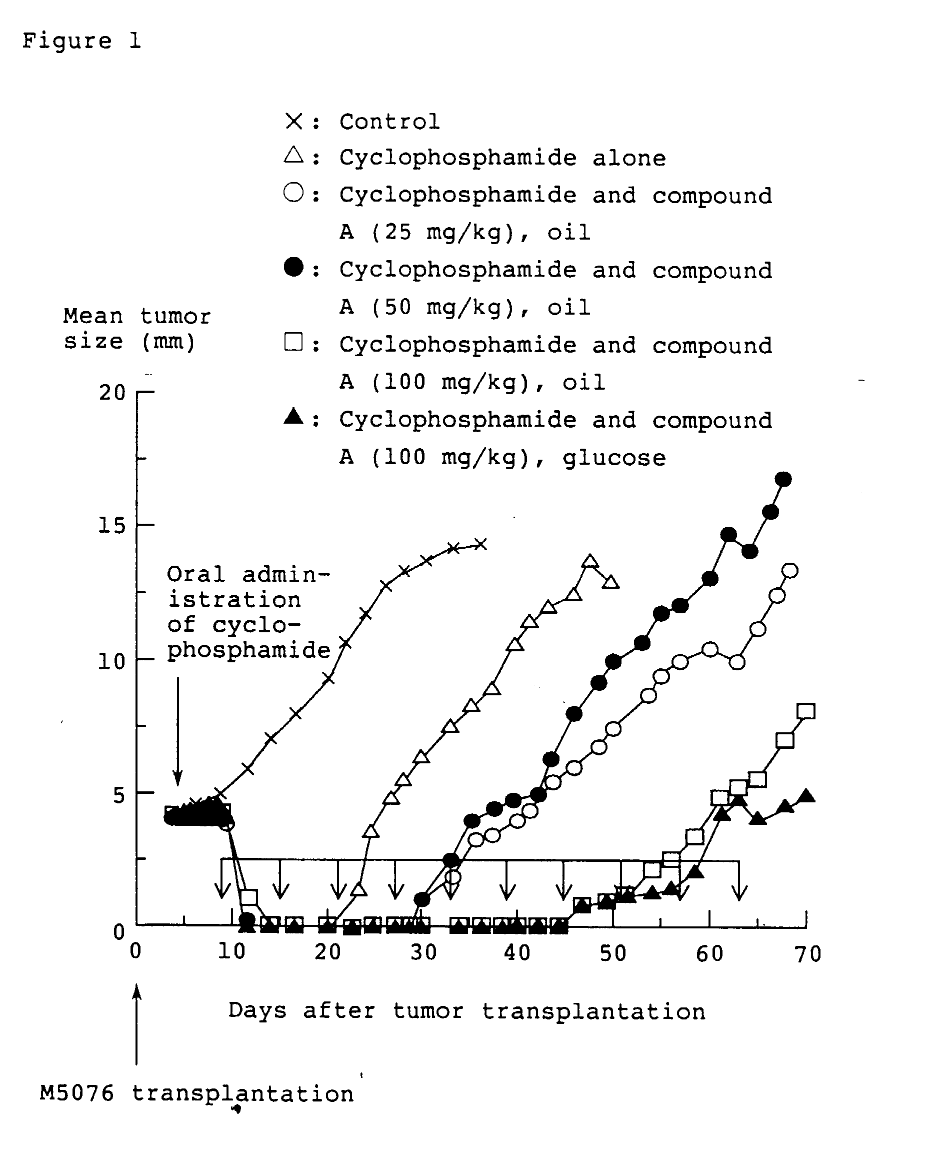

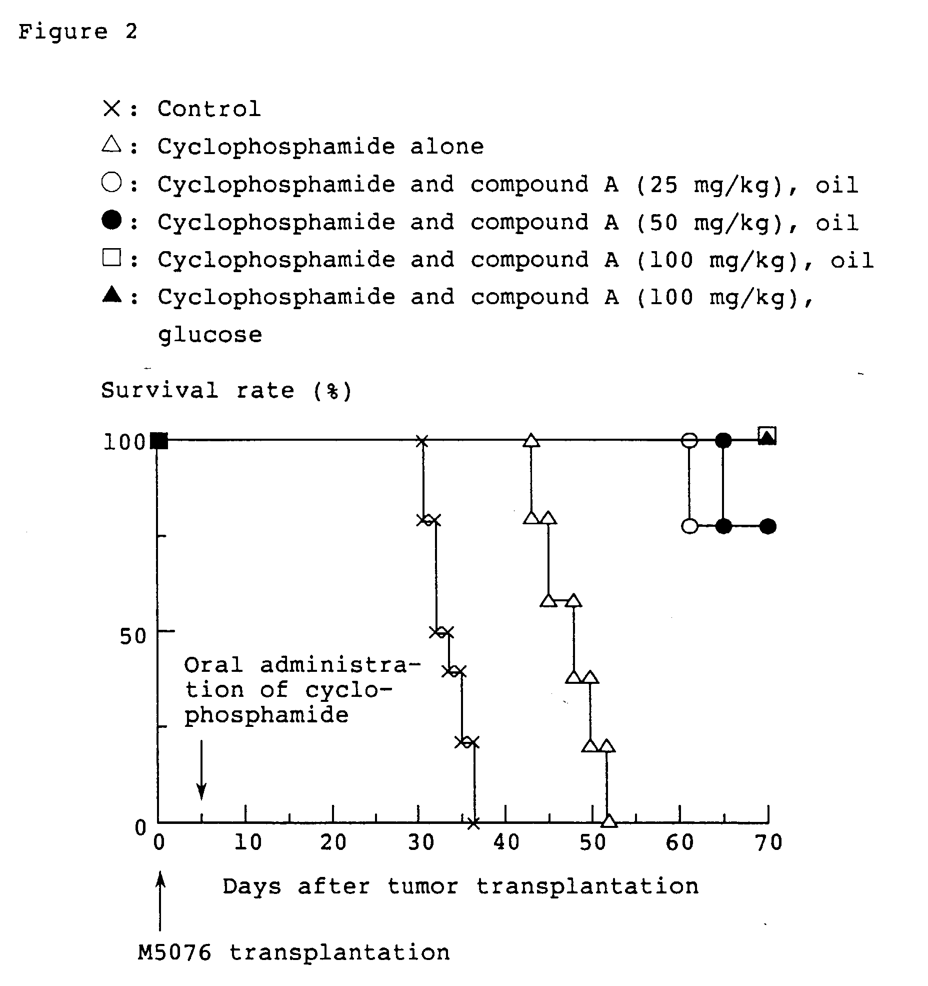

As shown in Figure 1, tumors shrank to immeasurable size until 20 days after administration in the group receiving cyclophosphamide alone but thereafter grew again. In the group receiving compound A, the tumor shrinking duration was extended dose dependently. In the group receiving the β-cyclodextrin clathrate at 100 mg/kg, tumor shrinking remained at an immeasurable level in 2 of the 5 animals at 70 days after tumor cell transplantation. These results demonstrate which compound A extends the duration of remission and suppresses tumor recurrence.

Regarding animal survival time, all mice in the control group had died by 36 days after tumor cell transplantation but survival was extended by cyclophosphamide administration; in the group receiving compound A after cyclophosphamide administration, survival time was further extended (Figure 2). In the group receiving compound A, no deaths were noted until 60 days after tumor growth. Even at 70 days after transplantation, all animals survived in the group receiving the maximum dose. These results demonstrate which compound A enhances the effect of cyclophosphamide administration, extends remission time, and extends survival time.

### Experimental Example 2

Compound A as an inclusion formulation with maltosyl-β-cyclodextrin dissolved in 5% glucose solution was administered once a week by subcutaneous injection. Cisplatin dissolved in saline was intravenously injected once on the 5th day of tumor implantation.

5 x 10⁻⁵ cells of mouse reticulum cell sarcoma was intradermally implanted into female mice of C57BL/6 (Japan CLEA). On the 5th day of the implantation, 7.5 mg/kg of cisplatin was intravenously injected once. Compound A was administered once a week by subcutaneous injection from the 5th day of the implantation. Numbers of mice consist of 10 for the control and 5 for the treated groups.

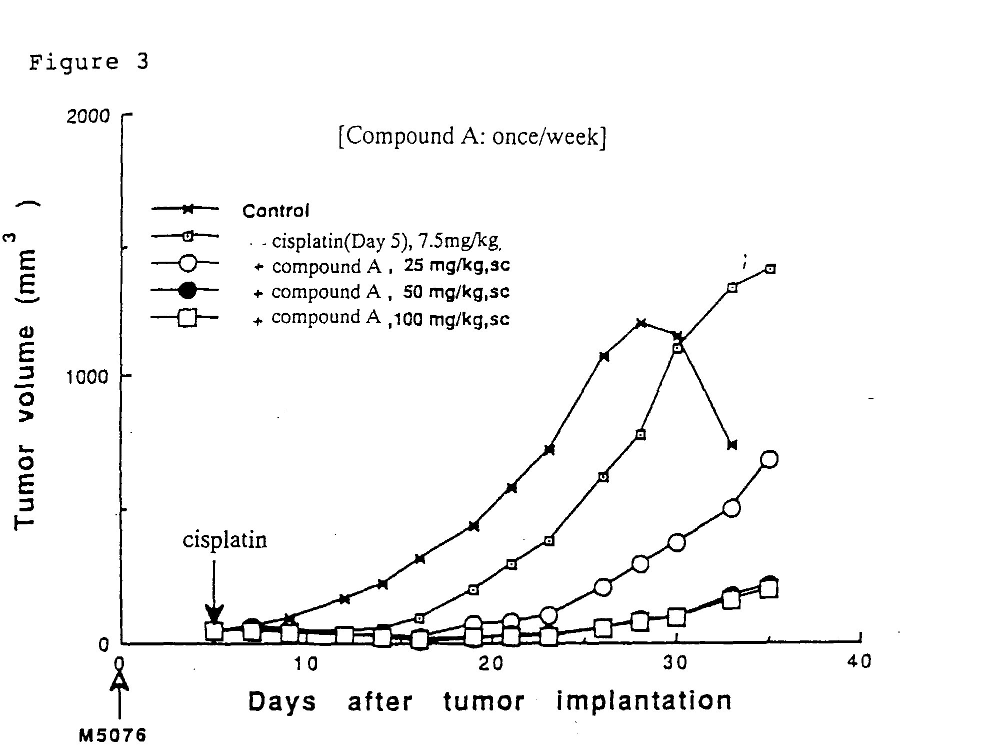

As shown in the Figure 3, the treatment with cisplatin alone exerted the cease of tumor growth, and followed by regrowth at the similar growth rate. The delay of the tumor growth is about 5 days at most. In the groups treated with compound A, once a week, along with single injection of cisplatin, inhibition of tumor growth was marked. Especially in the groups treated with 50 or 100 mg/kg of the compound A, tumor regression was observed and about half of the mice had no measurable tumors on the 10th day of the implantation. Later, regrowth was observed in each group; combinatory effect of compound A to the group of mice treated with cisplatin alone was evident. Further, liver metastasis was observed in the control and cisplatin-treated mice, but not in the mice treated with both cisplatin and compound A. The result indicates that compound A enhances or potentiates of antitumor activity of other treatments, and increases the term of response, or responsive rate (stable, partial response or complete response), or inhibiting the recurrence or metastasis.

## Claims

1. Use of an angiogenesis inhibitor for the manufacture of a medicament for inhibiting tumor recurrence.

2. The use according to claim 1, wherein the angiogenesis inhibitor is a fumagillol derivative or a salt thereof.

3. The use according to claim 2, wherein the fumagillol derivative is a compound represented by the formula: 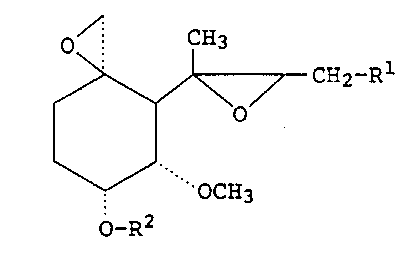 wherein R¹ represents a hydrocarbon group which may be substituted; R² represents a hydrocarbon group which may be substituted or an acyl group which may be substituted.

4. The use according to claim 3, wherein R¹ is an aliphatic hydrocarbon group which may be substituted.

5. The use according to claim 3, wherein R¹ is an alkyl group which may be substituted or an alkenyl group which may be substituted.

6. The use according to claim 3, wherein R¹ is a 2-methylpropyl group which may be substituted or a 2-methyl-1-propenyl group which may be substituted.

7. The use according to claim 3, wherein R² is an acyl group which may be substituted.

8. The use according to claim 3, wherein R² is a carbamoyl group which may be substituted.

9. The use according to claim 3, wherein R¹ is an alkyl group which may be substituted or an alkenyl group which may be substituted and wherein R² is an acyl group which may be substituted.

10. The use according to claim 9, wherein R² is an alkanoyl group which may be substituted, an alloyl group which may be substituted, a heterocyclic carbonyl group which may be substituted, a carbamoyl group which may be substituted or a thiocarbamoyl group which may be substituted.

11. The use according to claim 9, wherein R¹ is a 2-methylpropyl group or a 2-methyl-1-propenyl group.

12. The use according to claim 9, wherein R² is a carbamoyl group which may be substituted.

13. The use according to claim 9, wherein R² is a carbamoyl group substituted by monochloroacetyl or dichloroacetyl.

14. The use according to claim 1, wherein the angiogenesis inhibitor is 6-O-(N-chloroacetylcarbamoyl)fumagillol or a salt thereof.

15. A pharmaceutical composition comprising an angiogenesis inhibitor and cyclophosphamide.

16. Use of a combination of an angiogenesis inhibitor and cyclophosphamide for the manufacture of a medicament for use in combinational therapy for inhibiting tumor metastasis or recurrence.

17. The use according to claim 16, which is characterized by the additional combination with cisplatin and/or fluorouracil.

18. Use of a combination of an angiogenesis inhibitor and cisplatin for the manufacture of a medicament for inhibiting tumor recurrence.

19. Use of an angiogenesis inhibitor for the manufacture of a medicament for tumor treatment characterized by repeated administration of said angiogenesis inhibitor following administration of one or more tumor chemotherapeutic agents.

20. The use according to claim 18, wherein the tumor chemotherapeutic agent is cyclophosphamide.

21. Use of an angiogenesis inhibitor for the manufacture of a medicament for tumor treatment characterized by administration of said angiogenesis inhibitor following surgery, radiotherapy, thermotherapy, cryotherapy and/or laser cauterization therapy.

22. The use according to claim 21 intended for long-term repeated administration.

23. Use of an angiogenesis inhibitor for the manufacture of a medicament for enhancement of the antitumor activity, tumor metastasis-inhibiting activity or tumor recurrence-inhibiting activity of another tumor chemotherapeutic agent.

24. Use of 6-O-(N-chloroacetylcarbamoyl)fumagillol or a salt thereof for the manufacture of a medicament for use in the treatment of tumors selected from tumor of brain, tumor of breast, tumor of uterine cervix, tumor of pancreas, tumor of kidney, leukemia, tumor of colon, tumor of kaposi sarcoma, tumor of melanoma.

25. Use of 6-O-(N-chloroacetylcarbamoyl)fumagillol or a salt thereof for the manufacture of a medicament for use in treating a tumor by inhibiting tumor metastasis to brain.
